# EUROPEAN PATENT SPECIFICATION

(11) **EP 2 268 221 B1**
(45) Date of publication and mention of the grant of the patent: **03.10.2018**
(21) Application number: 09727303.1
(22) Date of filing: 31.03.2009
(51) Int. Cl.: H01H 15/00, A61B 18/00, A61B 18/14

(54) **ELECTROSURGICAL PENCIL INCLUDING IMPROVED CONTROLS**
ELEKTROCHIRURGISCHER STIFT MIT VERBESSERTER STEUERUNG
CRAYON ÉLECTRO CHIRURGICAL INCLUANT DES COMMANDES AMÉLIORÉES

(30) Priority: 27.02.2009 US 394456; 23.06.2008 US 144352; 31.03.2008 US 40843 P; 31.03.2008 US 40836 P; 31.03.2008 US 40916 P; 31.03.2008 US 40938 P; 26.02.2009 US 393089; 23.06.2008 US 144372; 23.06.2008 US 144367; 23.06.2008 US 144356
(43) Date of publication of application: 05.01.2011
(73) Proprietor: Covidien LP, Mansfield, MA 02048 (US)
(72) Inventor: FRY, Monte, Longmont CO 80501 (US); KERR, Duane, Loveland CO 80537 (US); RESCHKE, Arlan, J., Longmont CO 80501 (US); HEARD, David, N., Boulder CO 80303 (US); ALLEN, James, D., Broomfield CO 80020 (US); HORNER, Glenn, A., Boulder, CO 80304 (US)
(74) Representative: Soames, Candida Jane
(86) International application number: PCT/US2009/038980
(87) International publication number: WO 2009/124063

(56) References cited:
- EP-A- 1 852 078
- WO-A-01/39681
- GB-A- 2 257 831
- JP-A- 2005 026 166
- US-A1- 2005 067 264
- US-A1- 2006 178 667
- US-B1- 6 333 479

## Description

### CROSS REFERENCE TO RELATED APPLICATION

### BACKGROUND

### Technical Field

The present disclosure relates generally to electrosurgical instruments and, more particularly, to an electrosurgical pencil having a plurality of hand-accessible variable controls.

### Background of Related Art

Electrosurgical instruments have become widely used by surgeons in recent years. Accordingly, a need has developed for equipment and instruments which are easy to handle, are reliable and are safe in an operating environment. By and large, most electrosurgical instruments are hand-held instruments, e.g., an electrosurgical pencil, which transfer radio-frequency (RF) electrical or electrosurgical energy to a tissue site. The electrosurgical energy is returned to the electrosurgical source via a return electrode pad positioned under a patient (i.e., a monopolar system configuration) or a smaller return electrode positionable in bodily contact with or immediately adjacent to the surgical site (i.e., a bipolar system configuration). The waveforms produced by the RF source yield a predetermined electrosurgical effect known generally as electrosurgical cutting and fulguration.

As used herein the term "electrosurgical pencil" is intended to include instruments which have a handpiece which is attached to an active electrode and which is used to cauterize, coagulate and/or cut tissue. Typically, the electrosurgical pencil may be operated by a handswitch or a foot switch. The active electrode is an electrically conducting element which is usually elongated and may be in the form of a thin flat blade with a pointed or rounded distal end. Alternatively, the active electrode may include an elongated narrow cylindrical needle which is solid or hollow with a flat, rounded, pointed or slanted distal end. Typically electrodes of this sort are known in the art as "blade", "loop" or "snare", "needle" or "ball" electrodes.

As mentioned above, the handpiece of the electrosurgical pencil is connected to a suitable electrosurgical energy source (i.e., generator) which produces the radio-frequency electrical energy necessary for the operation of the electrosurgical pencil. In general, when an operation is performed on a patient with an electrosurgical pencil, electrical energy from the electrosurgical generator is conducted through the active electrode to the tissue at the site of the operation and then through the patient to a return electrode. The return electrode is typically placed at a convenient place on the patient's body and is attached to the generator by a conductive material. Typically, the surgeon activates the controls on the electrosurgical pencil to select the modes/waveforms to achieve a desired surgical effect.

The power or energy parameters are typically controlled from outside the sterile field which requires an intermediary like a circulating nurse to make such adjustment.

A typical electrosurgical generator has numerous controls for selecting an electrosurgical output. For example, the surgeon can select various surgical "modes" to treat tissue: cut, blend (blend levels 1-3), low cut, desiccate, fulgurate, spray, etc. The surgeon also has the option of selecting a range of power settings typically ranging from 1-300W. As can be appreciated, this gives the surgeon a great deal of variety when treating tissue. However, so many options also tend to complicate simple surgical procedures and may lead to confusion. Moreover, surgeons typically follow preset control parameters and stay within known modes and power settings. Therefore, there exists a need to allow the surgeon to selectively control and easily select and regulate the various modes and power settings utilizing simple and ergonomically friendly controls associated with the electrosurgical pencil.

Existing electrosurgical instrument systems allow the surgeon to change between two pre-configured settings (i.e., coagulation and cutting) via two discrete switches disposed on the electrosurgical pencil itself. Other electrosurgical instrument systems allow the surgeon to increment the power applied when the coagulating or cutting switch of the instrument is depressed by adjusting or closing a switch on the electrosurgical generator. The surgeon then needs to visually verify the change in the power being applied by looking at various displays and/or meters on the electrosurgical generator. In other words, all of the adjustments to the electrosurgical instrument and parameters being monitored during the use of the electrosurgical instrument are typically located on the electrosurgical generator. As such, the surgeon must continually monitor the electrosurgical generator during the surgical procedure. Furthermore, someone outside the sterile field must continually adjust the parameters of the electrical instrument, which prolongs the duration of the procedure.

Accordingly, the need exists for electrosurgical instruments which do not require the surgeon to continually monitor the electrosurgical generator during the surgical procedure. Further, a need exists for electrosurgical instruments, which permit the surgeon to accurately self-adjust the electrical parameters of the instrument from within the sterile field. In addition, the need exists for electrosurgical instruments which may be configured such that the power output can be adjusted without the surgeon having to turn his/her vision away from the operating site and toward the electrosurgical generator.

US 2006/0178667 A1 discloses an electrosurgical pencil selectively connectable to an electrosurgical generator. The electrosurgical pencil includes an elongated housing; at least one electrocautery end effector removably supportable within the housing and extending distally from the housing, the electrocautery end effector being connected to the electrosurgical generator; and at least one voltage divider network supported on the housing. The at least one voltage divider network is electrically connected to the electrosurgical generator and controls at least one of the intensity of electrosurgical energy being delivered to the electrosurgical pencil and the mode of electrosurgical energy being delivered to the electrosurgical pencil. The voltage divider network generates a plurality of characteristic voltages which are measurable by the electrosurgical generator and which electrosurgical generator in turn transmits a corresponding waveform duty cycle at a particular intensity to the electrocautery end effector of the electrosurgical pencil.

### SUMMARY

According to the present invention, an electrosurgical pencil is provided including an elongated housing configured to support an electrocautery electrode extending distally therefrom; at least one voltage divider network supported in the housing, the at least one voltage divider network operable to electrically connect to the source of electrosurgical energy for controlling an intensity of electrosurgical energy being delivered to the electrocautery electrode; and an intensity controller slidably supported on the housing, wherein the intensity controller is configured to exert a force on each of the housing and the at least one voltage divider network, wherein the intensity controller provides a tactile feedback to a user of the electrosurgical pencil as the intensity controller is moved relative to the housing.

The intensity controller includes a torsion spring pivotally supported on a body portion thereof, wherein the torsion spring is in contact with at least one of the housing and the electrical circuit.

The torsion spring includes a first leg configured for engagement with a tactile feature formed in the housing and a second leg configured for engagement with the at least one voltage divider network.

### BRIEF DESCRIPTION OF THE DRAWINGS

The accompanying drawings, which are incorporated in and constitute a part of this specification illustrate embodiments of the invention, and together with a general description of the invention given above, and the detailed description of the embodiments given below, serve to explain the principles of the invention.

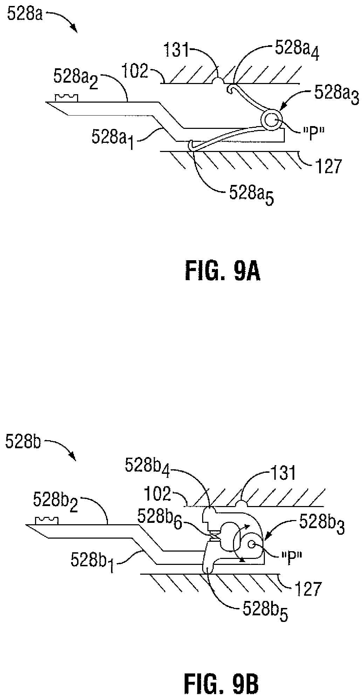

It is only Figure 9A and the associated description that provides an embodiment of all of the features of the present invention in combination and as claimed.
FIG. 1 is a perspective view of a prior ait electrosurgical system including an electrosurgical generator and an electrosurgical pencil;
FIG. 2 is an exploded perspective view of the electrosurgical pencil of FIG. 1 ;
FIG. 3 is a longitudinal, cross-sectional, side elevational view of the electrosurgical pencil of FIGS. 1 and 2;
FIG. 4 is an enlarged view of the indicated area of detail of FIG. 3;
FIG. 5 is an exploded perspective view of a voltage divider network;
FIG. 6A is a schematic side elevational view of a slider, which is not according to an embodiment of the present invention, for use in an electrosurgical pencil as shown in FIGS. 1-4;
FIG. 6B is a schematic side elevational view of a slider, which is not according to an embodiment of the present invention, for use in an electrosurgical pencil as shown in FIGS. 1-4;
FIG. 6C is a schematic side elevational view of a slider, which is not according to an embodiment of the present invention, for use in an electrosurgical pencil as shown in FIGS. 1-4;
FIG. 6D is a schematic perspective view, with parts separated, of a slider, which is not according to an embodiment of the present invention, for use in an electrosurgical pencil as shown in FIGS. 1-4;
FIG. 7A is a schematic side elevational view of an alternate slider, which is not according to an embodiment of the present invention, for use in an electrosurgical pencil as shown in FIGS. 1-4;
FIG. 7B is a schematic side elevational view of the alternate slider, which is not according to an embodiment of the present invention, for use in an electro surgical pencil as shown in FIGS. 1-4;
FIG. 7C is a schematic side elevational view of the alternate slider, which is not according to an embodiment of the present invention, for use in an electrosurgical pencil as shown in FIGS. 1-4;
FIG, 8A is a schematic illustration of a further alternate slider and a tactile mask, which is not according to an embodiment of the present invention, for use in an electrosurgical pencil as shown in FIGS. 1-4;
FIG. 8B is a schematic illustration of the further alternate slider according and a tactile mask, which is not according to an embodiment of the present invention, for use in an electrosurgical pencil as shown in FIGS. 1-4;
FIG. 9A is a schematic side elevational view of an alternate slider according to an embodiment of the present invention, for use in an electrosurgical pencil as shown in FIGS. 1-4; and
FIG. 9B is a schematic side elevational view of a further alternate slider, which is not according to an embodiment of the present invention, for use in an electrosurgical pencil as shown in FIGS. 1-4.

### DETAILED DESCRIPTION

A preferred embodiment of the presently disclosed electrosurgical pencil will now be described in detail with reference to the drawing figure 9A. In the figures like reference numerals identify similar or identical elements. As used herein, the term "distal" refers to that portion which is further from the user while the term "proximal" refers to that portion which is closer to the user or surgeon.

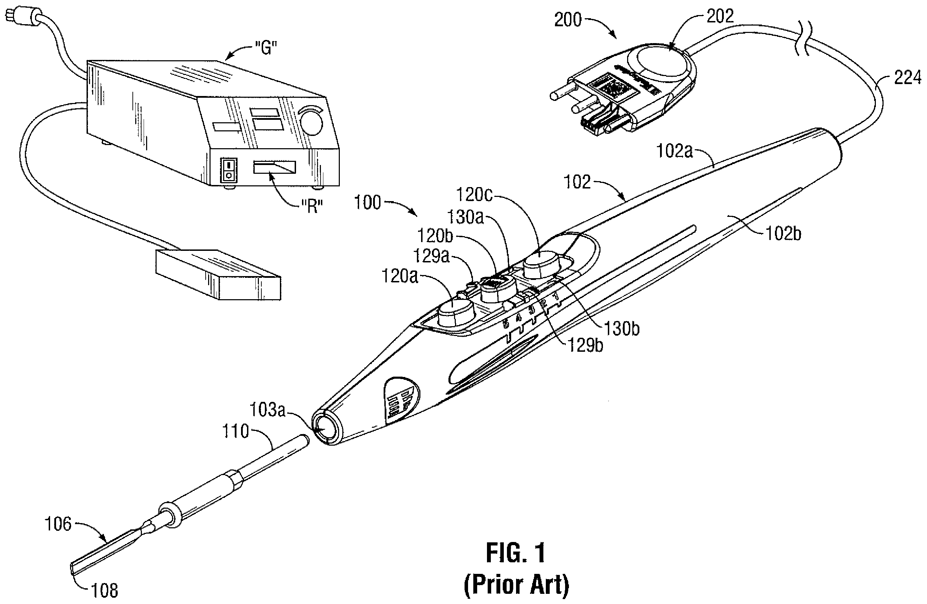

FIG. 1 sets forth a perspective view of an electrosurgical system including an electrosurgical pencil 100 constructed in accordance with a prior art embodiment. While the following description will be directed towards electrosurgical pencils it is envisioned that the features and concepts (or portions thereof) of the present disclosure can be applied to any electrosurgical type instrument, e.g., forceps, suction coagulates, vessel sealers, wands, etc.

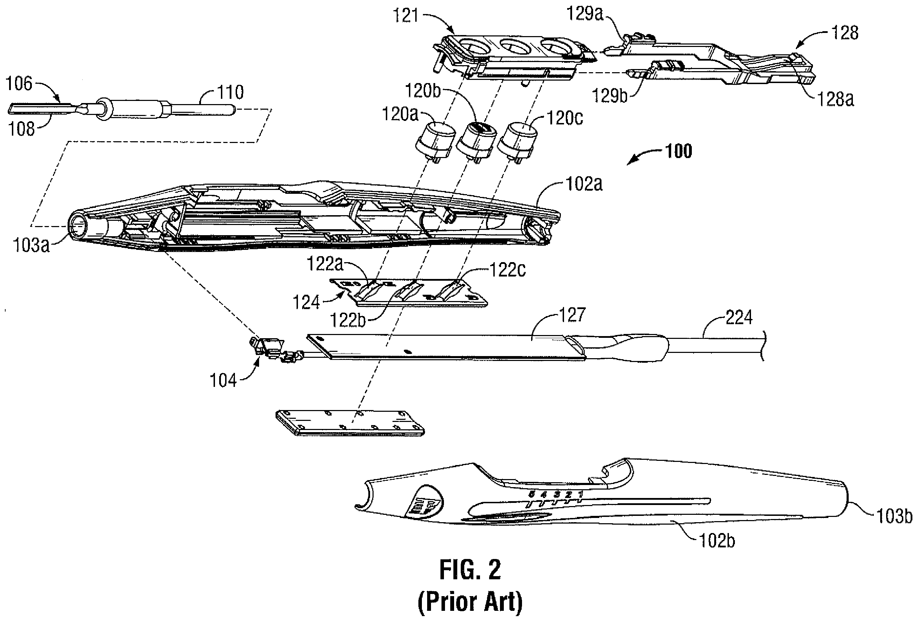

As seen in FIGS. 1-5, electrosurgical pencil 100 includes an elongated housing 102 having a right-half shell section 102a and a left-half shell section 102b. As seen in FIGS. 1 and 2, when right and left-half shell sections 102a, 102b are connected to one another, a distal opening 103a is defined therebetween, through which an electrode 106 extends, and a proximal opening 103b (see FIG. 2) is defined therebetween, through which connecting cable 224 (see FIG. 1) extends, As seen in FIG. 1, electrosurgical pencil 100 is coupled to an electrosurgical generator "G" via a plug assembly 200 connected to connecting cable 224.

As seen in FIG. 2, electrosurgical pencil 100 further includes an electrode receptacle 104 disposed at a distal end of housing 102, and a replaceable electrode 106 operatively and removably connectable to electrode receptacle 104.

With continued reference to FIGS. 1-3, electrosurgical pencil 100 includes three activation buttons 120a-120c, each of which is reciprocally supported in a carrier 121 (see FIG. 2) of a controller unit which is supported in housing 102. Each activation button 120a-120c includes a portion which extends through an upper surface of housing 102.

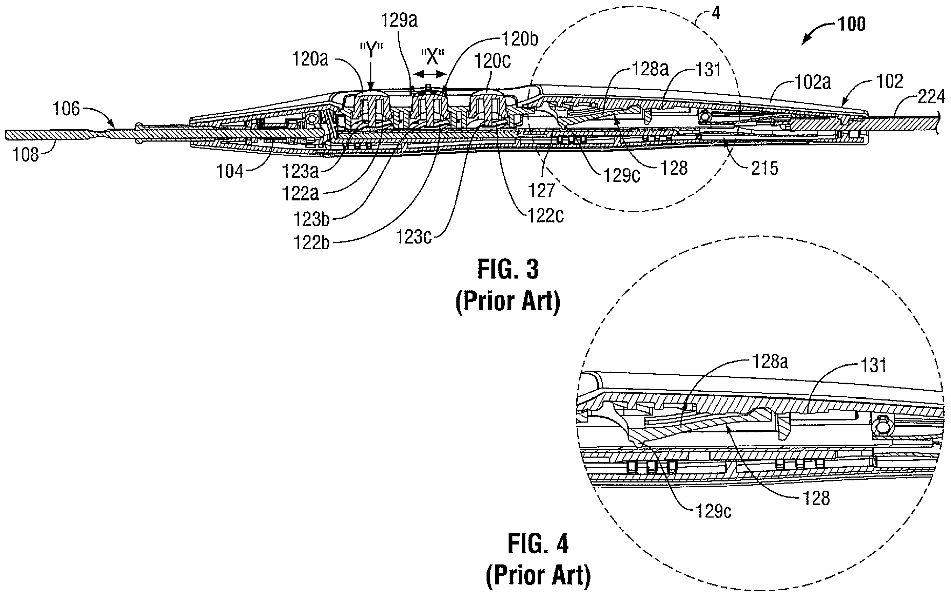

As seen in FIGS. 2 and 3, each activation button 120a-120c is operatively supported on a respective tactile element 122a-122c formed in a switch plate 124.

Each activation button 120a-120c controls the transmission of RF electrical energy supplied from generator "G" to electrode 106. Switch plate 124 is positioned over the top of a voltage divider network 127 (hereinafter "VDN 127") such that tactile elements 122a-122c are in operative association therewith.

As seen in FIGS. 1-4, electrosurgical pencil 100 includes an intensity controller 128 slidingly supported in housing 102. Intensity controller 128 includes a pair of nubs 129a, 129b which are slidingly supported, one each, in respective guide channels 130a, 130b (see FIG. 1).

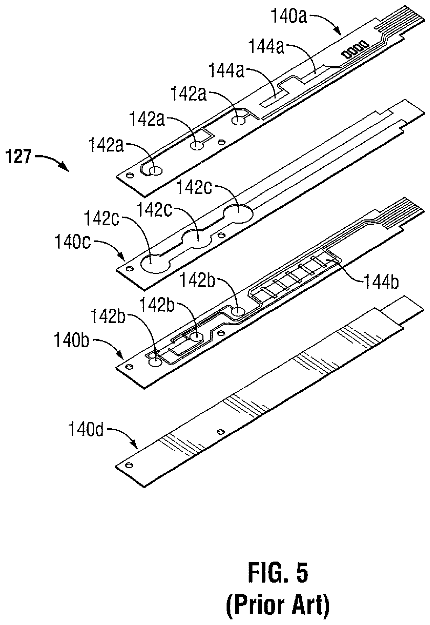

As seen in FIGS. 3 and 4, intensity controller 128 includes a third nub 129c extending from a bottom surface thereof which contacts and presses into or against VDN 127. As seen in FIG. 5, VDN 127 includes electrical contacts 144a provided on upper layer 140a and resistive element 144b on lower layer 140b. In this manner, as intensity controller 128 is displaced in a distal and proximal direction relative to housing 102, third nub 129c moves along VDN 127, thereby pressing electrical contact 144a from upper layer 140a of VDN 127 against resistance element 144b of lower layer 140b of VDN 127. In so doing, a resistance value of resistance element 144b is changed thereby changing the value of the voltage measured by electrosurgical generator "G". The electrosurgical generator "G" in turn varies the intensity of the waveform being transmitted to electrode 106.

Slidable manipulation or movement of intensity controller 128 adjusts the power parameters (e.g., voltage, power and/or current intensity) and/or the power verses impedance curve shape to affect the output intensity of the waveform.

In order to vary the intensity of the power parameters of electrosurgical pencil 100, the surgeon displaces intensity controller 128, by manipulating at least one of nubs 129a, 129b, in either of the directions indicated by double-headed arrow "X" (see FIG. 3).

Intensity controller 128 is also operable to provide a degree of tactile feedback by the inter-engagement of resilient finger 128a of intensity controller 128 in detents 131 formed along an inner surface of right-half shell section 102a (see FIGS. 3 and 4).

As seen in FIG. 5, VDN 127 includes a pair of layers 140a, 140b of resilient material each supporting a plurality of electrical contacts 142a, 142b thereon. Electrical contacts 142a from an upper layer 140a of VDN 127 are in juxtaposed electrical relation with respect to electrical contacts 142b from a lower layer 140b of VDN 127. The electrical contacts 142a, 142b of the upper and the lower layers 140a, 140b of VDN 127 are in juxtaposed relation with respective tactile elements 122a-122c.

Upper and lower layers 140a, 140b of VDN 127 are separated by a dividing layer 140c. Dividing layer 140c includes a first series of apertures 142c formed therein which are in vertical registration with electrical contacts 142a, 142b. Dividing layer 140c includes a second aperture 144c formed therein which is in vertical registration between electrical contacts 144a provided on upper layer 140a and a variable resistance element 144d provided on lower layer 140b. Upper layer 140a, lower layer 140b, and dividing layer 140c are supported on a support layer 140d.

In operation, and depending on the particular electrosurgical function desired, the surgeon depresses one of activation buttons 120a∼120c, in the direction indicated by arrow "Y" (see FIG. 3) thereby urging and/or deflecting a corresponding tactile element 122a- 122c against VDN 127 and thereby causing the respective electrical contact 142a of upper layer 140a to electrically engage the respective electrical contact 142b of the lower layer 140b. In so doing, a respective characteristic voltage is generated and measured by electrosurgical generator "G". In turn, depending on the characteristic voltage generated, generator "G" selects and transmits an appropriate waveform output to electrocautery blade 106,

Reference may be made to U.S. Application Serial No. 11/337,990 filed on January 24, 2006, corresponding to US publication No. 2006/0178667 A1, for a more detailed discussion of the construction and operation of electrosurgical pencil 100.

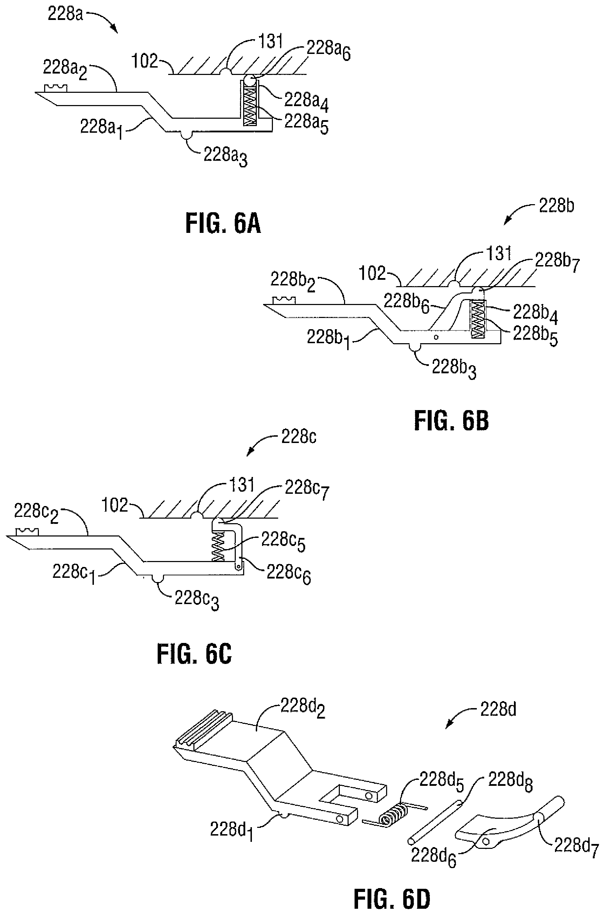

Turning now to FIGS. 6A-6D, a series of sliders or intensity controllers 228 is shown. Sliders 228 are configured to increase a contact force exerted on VDN 127 while maintaining a degree of facility for an end user to move slider 228 relative to housing 102 of electrosurgical pencil 100.

As seen in FIG. 6A, a slider 228a may include a body portion 228ai and at least one arm 228a2 extending from body portion 228ai and configured for slidable engagement in guide channels 130a, 130b (see FIG. 1) of electrosurgical pencil 100. Slider 228a includes a nub 228a3 extending or projecting from a bottom surface thereof, such as, for example, from a bottom surface of body portion 22Sa1. Slider 228a further includes a spring plunger assembly having a stem 22Sa4 extending from body portion 228aj, on a side opposite nub 228a3, and defining recess configured to retain a biasing member 228a₅ and an actuator 228a₆ therein. The spring plunger assembly is located distal or proximal of nub 228a₃.

In use, as slider 228a is moved distally and proximally relative to housing 102 of electrosurgical pencil 100, nub 228a₃ moves along VDN 127 thereby affecting VDN 127 while actuator 228a₆ of the spring plunger assembly inter-engages with detents or tactile features 131 formed in housing 102 of electrosurgical pencil 100 to thereby provide a degree of tactile feedback to the user of electrosurgical pencil 100. Biasing member 228a₅ functions to maintain nub 228a₃ in contact with VDN 127 and actuator 228a₆ of the spring plunger assembly in contact with detents or tactile features 131 formed in housing 102 of electrosurgical pencil 100.

As seen in FIG. 6B, a slider 228b may include a body portion 228b₁ and at least one arm 228b₂ extending from body portion 228b₁ and configured for slidable engagement in guide channels 130a, 130b (see FIG. 1) of electrosurgical pencil 100. Slider 228b includes a nub 228b₃ extending or projecting from a bottom surface thereof, such as, for example, from a bottom surface of body portion 228b₁. Slider 228b further includes a spring lever assembly having a stem 228b₄ extending from body portion 228b₁, on a side opposite nub 228b₃, and defining a recess configured to retain a biasing member 228b₅ therein. The spring lever assembly further includes a lever 228b₆ pivotally connected to body portion 228b₁ and having a tip 228b₇ configured to extend over or overlie biasing member 228b₅. The spring lever assembly is configured such that stem 228b₄ is located distal or proximal of nub 228b₃ and such that lever 228b₆ extends away from nub 228b₃.

In use, as slider 228b is moved distally and proximally relative to housing 102 of electrosurgical pencil 100, nub 228b₃ moves along VDN 127 thereby affecting VDN 127 while tip 228b₇ of lever 228b₆ of the spring lever assembly inter-engages with detents or tactile features 131 formed in housing 102 of electrosurgical pencil 100 to thereby provide a degree of tactile feedback to the user of electro surgical pencil 100. Biasing member 228b₅ functions to maintain nub 228b₃ in contact with VDN 127 and tip 228b₇ of lever 228b₆ of the spring lever assembly in contact with detents or tactile features 131 formed in housing 102 of electrosurgical pencil 100.

As seen in FIG. 6C, a slider 228c may include a body portion 228c₁ and at least one arm 228c₂ extending from body portion 228c₁ and configured for slidable engagement in guide channels 130a, 130b (see FIG. 1) of electrosurgical pencil 100. Slider 228c includes a nub 228c₃ extending or projecting from a bottom surface thereof, such as, for example, from a bottom surface of body portion 228c₁. Slider 228c further includes a spring lever assembly having a biasing member 228c₅ supported on body portion 228c₁, on a side opposite nub 228c₃, and a lever 228c₆ pivotally connected to body portion 228c₁ and having a tip 228c₇ configured to extend over or overlie biasing member 228c₅. The spring lever assembly is configured such that biasing member 228c₅ is located distal or proximal of nub 228c₃ and such that lever 228c₆ extends away from nub 228c₃.

In use, as slider 228c is moved distally and proximally relative to housing 102 of electrosurgical pencil 100, nub 228c₃ moves along VDN 127 thereby affecting VDN 127 while tip 228c₇ of lever 228c₆ of the spring lever assembly inter-engages with detents or tactile features 131 formed in housing 102 of electrosurgical pencil 100 to thereby provide a degree of tactile feedback to the user of electrosurgical pencil 100. Biasing member 228c₅ functions to maintain nub 228c₃ in contact with VDN 127 and tip 228c₇ of lever 228c₆ of the spring lever assembly in contact with detents or tactile features 131 formed in housing 102 of electrosurgical pencil 100.

In each of sliders 228a-228c shown in FIGS. 6A-6C and described above, it is contemplated that in some arrangements that actuator 228a₆, or tips 228b₇, 228c₇ of levers 228b₆ 228c₆ may axially overlie respective nubs 228a₃-228c₃. In this manner, the force of the biasing member 228a₅-228c₅ acts directly in line with respective nubs 228a₃-228c₃.

Although in FIGS. 6B-6C it is shown to a use coil spring as the biasing member, it is contemplated that these slider designs may alternatively incorporate torsion springs of the type shown in FIG 6D. As seen in FIG. 6D, a slider 228d may include a body portion 228d₁ and at least one arm 228d₂ extending from body portion 228d₁ and configured for slidable engagement in guide channels 130a, 130b (see FIG. 1) of electrosurgical pencil 100. Slider 228d includes a nub 228d₃ extending or projecting from a bottom surface thereof, such as, for example, from a bottom surface of body portion 228d₁. Slider 228d further includes a torsion spring lever assembly supported on body portion 228d₁ having a biasing member 228d₅ and a connector rod 228d₅ pivotally connecting lever 228d₆ to body portion 228d₁ on a side adjacent nub 228d₃. Lever 228d₆ includes a tip 228d₇ configured such that biasing member 228d₅ is located distal or proximal of nub 228d₃.

In use, as slider 228d is moved distally and proximally relative to housing 102 of electrosurgical pencil 100, nub 228d₃ moves along VDN 127 thereby affecting VDN 127 while tip 228d₇ of lever 228d₆ of the spring lever assembly inter-engages with detents or tactile features 131 formed in housing 102 of electrosurgical pencil 100 to thereby provide a degree of tactile feedback to the user of electrosurgical pencil 100. Biasing member 228d₅ functions to maintain nub 228d₃ in contact with VDN 127 and tip 228d₇ of lever 228d₆ of the torsion spring lever assembly in contact with detents or tactile features 131 formed in housing 102 of electrosurgical pencil 100. One advantage to using a torsion spring lever assembly configuration as set forth in FIG. 6D is that such a configuration provides greater spring deflections with smaller spring constants, thus making the delivered force less sensitive to dimensional variations in slider 228d.

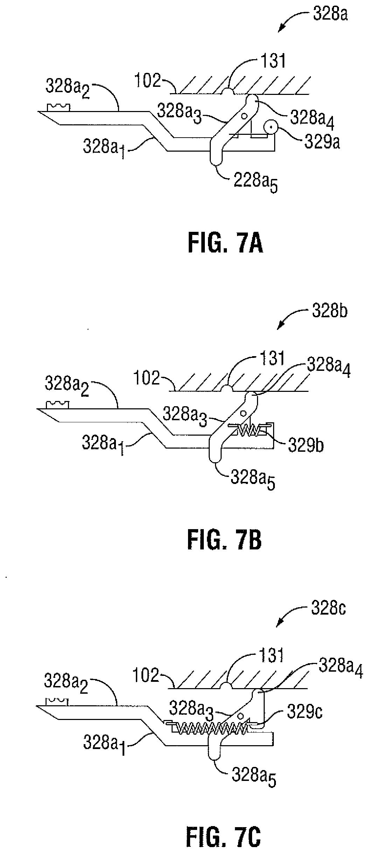

Turning now to FIGS. 7A-7C, a series of sliders or intensity controllers 328 is shown. Sliders 328 are configured to increase a contact force exerted on VDN 127 while maintaining a degree of facility for an end user to move slider 328 relative to housing 102 of electrosurgical pencil 100,

As seen in FIGS. 7A-7C, a slider 328a may include a body portion 328ai and at least one arm 32Sa₂ extending from body portion 328a] and configured for slidable engagement in guide channels 130a, 130b (see FIG. 1) of electrosurgical pencil 100. Slider 328a includes a lever 328a₃ pivotally connected to body portion 328a₁. Lever 328a₃ includes a first end 32Sa₄ configured to extend above body portion 32Sa₁ and a second end 328a₅ configured to extend below body portion 328a₁. First end 32Sa₄ of lever 328a₃ is configured to selectively engage detents or tactile features 131 formed in housing 102 of electrosurgical pencil 100 and second end 328a₅ of lever 328a₃ is configured to selectively engage VDN 127.

As seen in FIG. 7A, slider 328a may include a biasing member in the form of a coil or constant force spring 329a, or as seen in FIG. 7B slider 328a may include a biasing member in the form of a tensile spring 329b, or as seen in FIG. 7C slider 328a may include a biasing member in the form of a compression spring 329c. Biasing members 329a-329c are each configured or arranged so as to maintain first end 328a₄ of lever 328a₃ in contact with or in engagement with detents or tactile features 131 formed in housing 102 of electrosurgical pencil 100 and to maintain second end 328a₅ of lever 328a₃ in engagement with VDN 127. Biasing members 329a-329c may be secured to and extend between a suitable location on lever 328a₃ and a suitable location on body portion 32Sa₁.

In use, as slider 328a is moved distally and proximally relative to housing 102 of electrosurgical pencil 100, first end 328a₄ of lever 328a₃ inter-engages with detents or tactile features 131 formed in housing 102 of electro surgical pencil 100 to thereby provide a degree of tactile feedback to the user of electrosurgical pencil 100 while second end 328a₅ of lever 328a₃ moves along VDN 127 thereby affecting VDN 127. In particular, as first end 328a₄ of lever 328a₃ moves from one detent or tactile features 131 to an adjacent detent or tactile features 131, first end 328a₄ of lever 328a₃ is moved towards body portion 328a₁ and second end 328a₅ of lever 328a₃ moves off of or reduces a pressure on VDN 127 and also is moved towards body portion 328a₁. As first end 328a₄ of lever 328a₃ is moved into the adjacent detent or tactile features 131 second end 328a₅ of lever 328a₃ substantially strikes down onto, imparts or otherwise increases a pressure on VDN 127.

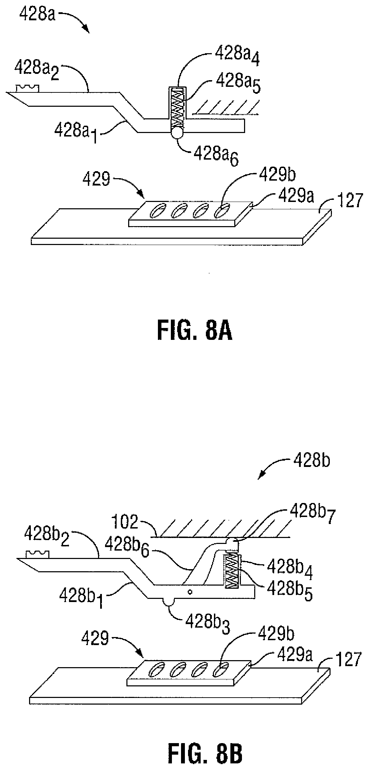

Turning now to FIGS. 8A and 8B₅ a series of sliders or intensity controllers 428 and a tactile mask 429 are shown. Sliders 428 are configured to increase a contact force exerted on VDN 127 while maintaining a degree of facility for an end user to move slider 428 relative to housing 102 of electrosurgical pencil 100. Tactile mask 429 is configured to cause slider 428 to impact or strike against VDN 127.

As seen in FIG. 8A, a slider 428a may include a body portion 428a₁ and at least one arm 428a₂ extending from body portion 428a₁ and configured for slidable engagement in guide channels 130a, 130b (see FIG. 1) of electrosurgical pencil 100. Slider 428a includes a spring plunger assembly having a stem 428a₄ extending from body portion 428a₁ and defining a recess configured to retain a biasing member 428a₅ and a tactile feedback transmitting feature in the form of an actuator 428a₆ therein. The spring plunger assembly is configured such that actuator 428a₆ extends from a bottom surface of body portion 428a₁, in the direction of VDN 127.

Tactile mask 429 includes an elongate body portion 429a configured to overlie VDN 127. Body portion 429a defines a plurality of apertures or windows 429b formed therein along a length thereof. Tactile mask 429 is positioned over VDN 127 at a location such that apertures 429b may align or register with variable resistance elements 144d provided on lower layer 140b of VDN 127 (see FIG. 5).

In use, as slider 428a is moved distally and proximally relative to housing 102 of electrosurgical pencil 100, actuator 428a₆ of spring plunger assembly moves over and between apertures 429b formed in tactile mask 429. In so doing, actuator 428a₆ of spring plunger assembly impacts or strikes against VDN 127. Additionally, the inter-engagement of actuator 428a₆ of spring plunger assembly with apertures 429b formed in tactile mask 429 provides a degree of tactile feedback to the user of electrosurgical pencil 100.

As seen in FIG. 8B, a slider 428b may include a body portion 428b₁ and at least one arm 428b₂ extending from body portion 428b₁ and configured for slidable engagement in guide channels 130a, 130b (see FIG. 1) of electrosurgical pencil 100. Slider 428b includes a tactile feedback transmitting feature in the form of a nub 428b₃ extending or projecting from a bottom surface thereof, such as, for example, from a bottom surface of body portion 428b₁. Slider 428b further includes a spring lever assembly having a stem 428b₄ extending from body portion 428b₁, on a side opposite nub 428b₃, and defining a recess configured to retain a biasing member 428b₅ therein. The spring lever assembly further includes a lever 428b₆ pivotally connected to body portion 428b₁ and having a tip 428b₇ configured to extend over or overlie biasing member 428b₅, The spring lever assembly is configured such that stem 428b₄ is located distal or proximal of nub 428b₃ and such that lever 428b₆ extends away from nub 428b₃.

In use, as slider 428b is moved distally and proximally relative to housing 102 of electrosurgical pencil 100, nub 428b₃ of slider 428b moves over and between apertures 429b formed in tactile mask 429. In so doing, nub 428b₃ of slider 428b contacts VDN 127. Additionally, the inter-engagement of nub 428b₃ of slider 428b with apertures 429b formed in tactile mask 429 provides a degree of tactile feedback to the user of electrosurgical pencil 100. Moreover, tip 428b₇ of lever 428b₆ rides against an inner surface of housing 102 of pencil 100 and biasing member 428b₅ act on tip 428b₇ of lever 428b₆ to exert a force on body portion 428b₁ and thereby press nub 428b₃ of slider 428b against tactile mask 429.

Tactile mask 429 may be constructed from a rigid, semi-rigid or non-rigid material, from a resilient or non-resilient material, from a conductive or non- conductive material, from any combination thereof, or from any material suitable for the intended purpose of defining apertures and transmitting forces through said apertures.

Turning now to FIGS. 9A and 9B, a series of sliders or intensity controllers 528 is shown. Sliders 528 are configured to increase a contact force exerted on VDN 127 while maintaining a degree of facility for an end user to move slider 528 relative to housing 102 of electrosurgical pencil 100.

As seen in FIG, 9A, a slider 528a may include a body portion 528a₁ and at least one arm 528a₂ extending from body portion 528a₁ and configured for slidable engagement in guide channels 130a, 130b (see FIG. 1) of electrosurgical pencil 100. Slider 528a includes a biasing member, in the form of a torsion spring 528a₃ pivotally supported on body portion 528a₁ at pivot point "P". Torsion spring 528a₃ includes a first leg 528a₄ extending from pivot point "P" and configured to engage a surface of housing 102 of electrosurgical pencil 100, and a second leg 528a₅ extending from pivot point "P" and configured to engage VDN 127. As seen in FIG. 9A, first leg 528a₄ of torsion spring 528a₃ extends above body portion 528a₁ and second leg 528a₅ of torsion spring 528a₃ extends below body portion 528a₁.

In use, as slider 528a is moved distally and proximally relative to housing 102 of electrosurgical pencil 100, second leg 528a₅ of torsion spring 528a₃ moves along VDN 127 thereby affecting VDN 127 while first leg 528a₄ of torsion spring 528a₃ inter-engages with detents or tactile features 131 formed in housing 102 of electrosurgical pencil 100 to thereby provide a degree of tactile feedback to the user of electrosurgical pencil 100. As first leg 528a₄ of torsion spring 528a₃ is flexed downwardly, in the direction of body portion 528a₁, as slider 528a is moved distally and proximally relative to housing 102 of electrosurgical pencil 100, second leg 528a₅ of torsion spring 528a₃ is pressed more or less into the surface of VDN 127.

As seen in FIG. 9B, a slider 528b may include a body portion 528b₁ and at least one arm 528b₂ extending from body portion 528b₁ and configured for slidable engagement in guide channels 130a, 130b (see FIG. 1) of electrosurgical pencil 100. Slider 528b includes a link assembly 528b₃ pivotally supported on body portion 528b₁ at pivot point "P". Link assembly 528b₃ includes a first leg 528b₄ extending from pivot point "P" and configured to engage a surface of housing 102 of electrosurgical pencil 100, a second leg 528b₅ extending from pivot point "P" and configured to engage VDN 127, and a biasing member 528b₆ interposed between first leg 528b₄ a second leg 528b₅. As seen in FIG, 9B, first leg 528b₄ of link assembly 528b₃ is in registration with or extends above second leg 528b₅ of link assembly 528b₃.

In use, as slider 528b is moved distally and proximally relative to housing 102 of electrosurgical pencil 100, second leg 528b₅ of link assembly 528b₃ moves along VDN 127 thereby affecting VDN 127 while first leg 528b₄ of link assembly 528b₃ inter-engages with detents or tactile features 131 formed in housing 102 of electrosurgical pencil 100 to thereby provide a degree of tactile feedback to the user of electrosurgical pencil 100. As first leg 528b₄ of link assembly 528b₃ is moved downwardly, in the direction of body portion 528b₁, as slider 528b is moved distally and proximally relative to housing 102 of electrosurgical pencil 100, biasing member 528b₆ transmits forces to second leg 528b₅ of link assembly 528b₃ to press more or less into the surface of VDN 127.

## Claims

1. An electrosurgical pencil (100), comprising:
an elongated housing (102) configured to support an electrocautery electrode (106) extending distally therefrom;
at least one voltage divider network (127) supported in the housing, the at least one voltage divider network operable to electrically connect to the source of electrosurgical energy for controlling an intensity of electrosurgical energy being delivered to the electrocautery electrode; and
an intensity controller (128, 528) slidably supported on the housing, wherein the intensity controller is configured to exert a force on each of the housing and the at least one voltage divider network, wherein the intensity controller provides a tactile feedback to a user of the electrosurgical pencil as the intensity controller is moved relative to the housing
**characterised in that**
the intensity controller includes a torsion spring (528a₃) pivotally supported on a body portion thereof, wherein the torsion spring is in contact with at least one of the housing and the electrical circuit, and
wherein the torsion spring includes a first leg (528a₄) configured for engagement with a tactile feature (131) formed in the housing and a second leg(528a₅) configured for engagement with the at least one voltage divider network.

2. The electrosurgical pencil of claim 1, wherein the intensity controller is a slider (528a) including the body portion (528a₁) and at least one arm (528a₂) extending from the body portion (528a₁) and configured for slidable engagement in at least one guide channel (130a, 130b) of the electrosurgical pencil (100).

3. The electrosurgical pencil of claim 2, wherein the slider includes arms (528a₂) extending from the body portion (528a₁) and configured for slidable engagement in guide channels (130a, 130b) of the electrosurgical pencil.

4. The electrosurgical pencil of claim 1, 2 or 3, wherein the first leg (528a₄) of the torsion spring (528a₃) extends above the body portion (528a₁) and the second leg (528a₅) of the torsion spring (528a₃) extends below the body portion (528a₁).

5. The electrosurgical pencil of any preceding claim configured such that as the intensity controller (528a) is moved distally and proximally relative to the housing (102) of the electrosurgical pencil (100), the second leg (528a₅) of the torsion spring (528a₃) moves along the voltage divider network (127) thereby affecting the voltage divider network while the first leg (528a₄) of the torsion spring (528a₃) inter-engages with tactile features (131) formed in the housing (102) of the electrosurgical pencil (100) to thereby provide a degree of tactile feedback to the user of the electrosurgical pencil (100).

6. The electrosurgical pencil of any preceding claim configured so that as the first leg (528a₄) of the torsion spring (528a₃) is flexed downwardly, in the direction of the body portion (528a₁), when the intensity controller (528a) is moved distally and proximally relative to the housing (102) of the electrosurgical pencil 100, the second leg (528a₅) of the torsion spring (528a₃) is pressed less into the surface of the voltage divider network (127).

7. The electrosurgical pencil of any preceding claim, comprising a plurality of tactile features including said tactile feature, wherein the tactile features are provided in the form of detents formed in the hosuing, wherein the intensity controller is a slider, configured so that as the slider (528a) is moved distally and proximally relative to the housing (102) of the electrosurgical pencil (100), the second leg (528a₅) of the torsion spring (528a₃) moves along the voltage divider network, VDN, (127) thereby affecting the VDN (127) while the first leg (528a₄) of the torsion spring (528a₃) inter-engages with the detents (131) formed in the housing (102) of the electrosurgical pencil (100) to thereby provide a degree of tactile feedback to the user of electrosurgical pencil (100), whereby when the first leg (528a₄) of the torsion spring (528a₃) is flexed downwardly to come out of engagement with a detent of the detents, in the direction of the body portion (528a₁), as the slider (528a) is moved distally and proximally relative to the housing (102) of the electrosurgical pencil (100), the second leg (528a₅) of the torsion spring (528a₃) is pressed less into the surface of the VDN (127).

## Patentansprüche

1. Elektrochirurgischer Stift (100), umfassend:
ein längliches Gehäuse (102), das konfiguriert ist, um eine Elektrokauterelektrode (106) zu tragen, die sich distal davon erstreckt;
mindestens ein Spannungsteilernetzwerk (127), das in dem Gehäuse aufgenommen ist, wobei das mindestens eine Spannungsteilernetzwerk ausgebildet ist, um elektrisch an die Quelle der elektrochirurgischen Energie angeschlossen zu werden, um die Energieintensität von elektrochirurgischer Energie zu regeln, die an die Elektrokauterelektrode geliefert wird; und
einen Intensitätsregler (128, 528), der gleitbar auf dem Gehäuse getragen wird, wobei der Intensitätsregler konfiguriert ist, um eine Kraft sowohl auf das Gehäuse als auch das mindestens eine Spannungsteilernetzwerk auszuüben, wobei der Intensitätsregler ein taktiles Feedback an einen Anwender des elektrochirurgischen Stifts liefert, wenn der Intensitätsregler in Bezug auf das Gehäuse bewegt wird,
**dadurch gekennzeichnet, dass** der Intensitätsregler eine Drehfeder (528a₅) enthält, die drehbar auf einem Körperabschnitt desselben montiert ist, wobei die Drehfeder in Kontakt mit mindestens einem von Gehäuse und elektrischem Schaltkreis steht, und
wobei die Drehfeder einen ersten Schenkel (528a₄) enthält, der für den Eingriff mit einer taktilen Vorrichtung (131) konfiguriert ist, die im Gehäuse ausgebildet ist, und einen zweiten Schenkel (528a₅), der für den Eingriff mit mindestens einem Spannungsteilernetzwerk konfiguriert ist.

2. Elektrochirurgischer Stift nach Anspruch 1, wobei der Intensitätsregler ein Schieber (528a) ist, der den Körperabschnitt (528a₁) und mindestens einen Arm (528a₂) umfasst, der sich aus dem Körperabschnitt (528a₁) erstreckt und für das gleitbare Einrasten in mindestens einen Führungskanal (130a, 130b) des elektrochirurgischen Stifts (100) konfiguriert ist.

3. Elektrochirurgischer Stift nach Anspruch 2, wobei der Schieber Arme (528a₂) enthält, die sich aus dem Körperabschnitt (528a₁) erstrecken und für das gleitbare Einrasten in Führungskanäle (130a, 130b) des elektrochirurgischen Stifts konfiguriert sind.

4. Elektrochirurgischer Stift nach Anspruch 1, 2 oder 3, wobei der erste Schenkel (528a₄) der Drehfeder (528a₃) sich über dem Körperabschnitt (528a₁) erstreckt und der zweite Schenkel (528a₅) der Drehfeder (528a₃) sich unter dem Körperabschnitt (528a₁) erstreckt.

5. Elektrochirurgischer Stift nach einem der vorstehenden Ansprüche, der so konfiguriert ist, dass, wenn der Intensitätsregler (528a) distal und proximal zu dem Gehäuse (102) des elektrochirurgischen Stifts (100) bewegt wird, der zweite Schenkel (528a₅) der Drehfeder (528a₅) sich entlang des Spannungsteilernetzwerks (127) bewegt, und damit das Spannungsteilernetzwerk beeinflusst, während der erste Schenkel (528a₄) der Drehfeder (528a₅) mit taktilen Einrichtungen (131) in Eingriff tritt, die im Gehäuse (102) des elektrochirurgischen Stifts (100) ausgebildet sind, um damit einen Grad von taktilem Feedback an den Anwender des elektrochirurgischen Stifts (100) zu liefern.

6. Elektrochirurgischer Stift nach einem der vorstehenden Ansprüche, der so konfiguriert ist, dass, wenn der erste Schenkel (528a₄) der Drehfeder (528a₃) in der Richtung des Körperabschnitts (528a₁) nach unten gebogen wird, wenn der Intensitätsregler (528a) distal und proximal in Bezug auf das Gehäuse (102) des elektrochirurgischen Stifts (100) bewegt wird, der zweite Schenkel (528as) der Drehfeder (528a₅) weniger in die Oberfläche des Spannungsteilernetzwerks (127) gedrückt wird.

7. Elektrochirurgischer Stift nach einem der vorstehenden Ansprüche, der mehrere taktile Einrichtungen umfasst, welche die taktile Einrichtung umfassen, wobei die taktilen Einrichtungen in der Form von Rastungen vorgesehen sind, die im Gehäuse ausgebildet sind, wobei der Intensitätsregler ein Schieber ist, der so konfiguriert ist, dass, wenn der Schieber (528a) distal und proximal in Bezug auf das Gehäuse (102) des elektrochirurgischen Stifts (100) bewegt wird, der zweite Schenkel (528a₅) der Drehfeder (528a₃) sich entlang des Spannungsteilernetzwerks, VDN, (127) bewegt, wodurch das VDN (127) beeinflusst wird, während der erste Schenkel (528a₄) der Drehfeder (528a₅) mit den im Gehäuse (102) des elektrochirurgischen Stifts (100) ausgebildeten Rastungen (131) in Eingriff tritt, um damit einen Grad von taktilem Feedback an den Anwender des elektrochirurgischen Stifts (100) zu liefern, wodurch, wenn der erste Schenkel (528a₄) der Drehfeder (528a₃) nach unten gebogen wird, um sich aus dem Eingriff mit einer Rastung der Rasterungen in der Richtung des Körperabschnitts (528a₁) zu lösen, wenn der Schieber (528a) distal und proximal in Bezug auf das Gehäuse (102) des elektrochirurgischen Stifts (100) bewegt wird, der zweite Schenkel (528a₅) der Drehfeder (528a₅) weniger in die Oberfläche des VDN (127) gedrückt wird.

## Revendications

1. Crayon électrochirurgical (100), comprenant :
un boîtier allongé (102) configuré pour supporter une électrode d'électrocautérisation (106) s'étendant distalement de celui-ci ;
au moins un réseau diviseur de tension (127) supporté dans le boîtier, l'au moins un réseau diviseur de tension étant actionnable pour être relié électriquement à la source d'énergie électrochirurgicale pour commander une intensité d'énergie électrochirurgicale qui est fournie à l'électrode d'électrocautérisation ; et
un dispositif de commande d'intensité (128, 528) supporté de manière coulissante sur le boîtier, dans lequel le dispositif de commande d'intensité est configuré pour exercer une force sur chacun du boîtier et de l'au moins un réseau diviseur de tension, dans lequel le dispositif de commande d'intensité fournit une rétroaction tactile à un utilisateur du crayon électrochirurgical à mesure que le dispositif de commande d'intensité est déplacé par rapport au boîtier,
**caractérisé en ce que**
le dispositif de commande d'intensité comprend un ressort de torsion (528a₅) supporté par pivotement sur une portion de corps de celui-ci, dans lequel le ressort de torsion est en contact avec au moins un parmi le boîtier et le circuit électrique, et
dans lequel le ressort de torsion comprend une première jambe (528a₄) configurée pour une mise en prise avec une caractéristique tactile (131) formée dans le boîtier et une deuxième jambe (528a₅) configurée pour une mise en prise avec l'au moins un réseau diviseur de tension.

2. Crayon électrochirurgical selon la revendication 1, dans lequel le dispositif de commande d'intensité est un coulisseau (528a) comprenant la portion de corps (528a₁) et au moins un bras (528a₂) s'étendant de la portion de corps (528a₁) et configuré pour la mise en prise coulissante dans au moins un canal de guidage (130a, 130b) du crayon électrochirurgical (100).

3. Crayon électrochirurgical selon la revendication 2, dans lequel le coulisseau comprend des bras (528a₂) s'étendant de la portion de corps (528a₁) et configuré pour une mise en prise coulissante dans des canaux de guidage (130a, 130b) du crayon électrochirurgical.

4. Crayon électrochirurgical selon la revendication 1, 2 ou 3, dans lequel la première jambe (528a₄) du ressort de torsion (528a₃) s'étend au-dessus de la portion de corps (528a₁) et la deuxième jambe (528a₅) du ressort de torsion (528a₃) s'étend sous la portion de corps (528a₁).

5. Crayon électrochirurgical selon l'une quelconque des revendications précédentes configuré de sorte que le dispositif de commande d'intensité (528a) est déplacé distalement et proximalement par rapport au boîtier (102) du crayon électrochirurgical (100), la deuxième jambe (528a₅) du ressort de torsion (528a₃) se déplace le long du réseau diviseur de tension (127) affectant ce faisant le réseau diviseur de tension pendant que la première jambe (528a₄) du ressort de torsion (528a₃) coopère avec des caractéristiques tactiles (131) formées dans le boîtier (102) du crayon électrochirurgical (100) pour fournir ce faisant un degré de rétroaction tactile à l'utilisateur du crayon électrochirurgical (100).

6. Crayon électrochirurgical selon l'une quelconque des revendications précédentes configuré de sorte que la première jambe (528a₄) du ressort de torsion (528a₃) est fléchie vers le bas, dans la direction de la portion de corps (528a₁), lorsque le dispositif de commande d'intensité (528a) est déplacé distalement et proximalement par rapport au boîtier (102) du crayon électrochirurgical (100), la deuxième jambe (528a₅) du ressort de torsion (528a₃) est pressée moins dans la surface du réseau diviseur de tension (127).

7. Crayon électrochirurgical selon l'une quelconque des revendications précédentes, comprenant une pluralité de caractéristiques tactiles comprenant ladite caractéristique tactile, dans lequel les caractéristiques tactiles sont prévues sous la forme de détentes formées dans le boîtier, dans lequel le dispositif de commande d'intensité est un coulisseau, configuré de sorte que le coulisseau (528a) est déplacé distalement et proximalement par rapport au boîtier (102) du crayon électrochirurgical (100), la deuxième jambe (528a₅) du ressort de torsion (528a₃) se déplace le long du réseau diviseur de tension, VDN, (127) affectant ce faisant le VDN (127) pendant que la première jambe (528a₄) du ressort de torsion (528a₃) coopère avec les détentes (131) formées dans le boîtier (102) du crayon électrochirurgical (100) pour fournir ce faisant un degré de rétroaction tactile à l'utilisateur du crayon électrochirurgical (100), dans lequel lorsque la première jambe (528a₄) du ressort de torsion (528a₃) est fléchie vers le bas pour se mettre hors prise d'une détente des détentes, dans la direction de la portion de corps (528a₁), à mesure que le coulisseau (528a) est déplacé distalement et proximalement par rapport au boîtier (102) du crayon électrochirurgical (100), la deuxième jambe (528a₅) du ressort de torsion (528a₃) est pressée moins dans la surface du VDN (127).
